# EUROPEAN PATENT SPECIFICATION

(11) **EP 4 197 354 B1**
(45) Date of publication and mention of the grant of the patent: **16.10.2024**
(21) Application number: 22213669.9
(22) Date of filing: 15.12.2022
(51) Int. Cl.: A24F 40/05, A24F 40/10

(54) **MICROPOROUS VAPORIZATION ASSEMBLY AND ELECTRONIC VAPORIZATION DEVICE**
MIKROPORÖSE VERDAMPFUNGSANORDNUNG UND ELEKTRONISCHE VERDAMPFUNGSVORRICHTUNG
ENSEMBLE DE VAPORISATION MICROPOREUX ET DISPOSITIF DE VAPORISATION ÉLECTRONIQUE

(30) Priority: 17.12.2021 CN 202111551849
(43) Date of publication of application: 21.06.2023
(73) Proprietor: Shenzhen Moore Vaporization Health & Medical Technology Co., Ltd., Shenzhen, Guangdong 518102 (CN)
(72) Inventor: OUYANG, Guanglin, Shenzhen (CN); ZHU, Yongxiong, Shenzhen (CN); TANG, Ming, Shenzhen (CN); LI, Lei, Shenzhen (CN)
(74) Representative: Michalski Hüttermann & Partner Patentanwälte mbB

(56) References cited:
- EP-A1- 3 251 531
- US-A1- 2019 216 135

## Description

### TECHNICAL FIELD

The present application relates to the field of vaporization technologies, and in particular, to a microporous vaporization assembly and an electronic vaporization device.

### BACKGROUND

An aerosol is a colloidal dispersion system formed by solid or liquid particles dispersing and suspending in a gaseous medium. The aerosol can be absorbed by human body through the respiratory system to provide users with a new alternative method of absorption. For example, a vaporization device, which can produce an aerosol by baking and heating an herbal or pasty aerosol-forming substrate, is applied in different fields to deliver an inhalable aerosol to users, replacing a conventional product form and a conventional absorption mode.

Generally, the vaporization device vaporizes the aerosol-forming substrate into an aerosol, and common vaporization methods include heating vaporization and ultrasonic vaporization. The principle of the ultrasonic vaporization includes that a microporous ultrasonic vaporization plate is driven to resonate by the high frequency vibration of a piezoceramic; vaporization holes in the microporous ultrasonic vaporization plate continuously and repeatedly deform or vibrate along with the vibration; and a solution is pressed and broken into fine droplets to form vaporized steam. In a microporous ultrasonic vaporization device, a vaporization plate is bonded to the piezoceramic plate. In a process of vaporization, the vaporization plate vibrates at a high frequency, and a liquid (which is not limited to a strong acid, a strong base, and a strong oxidant liquid) enters an adhesive layer between the vaporization plate and the piezoceramic plate due to vaporization vibration, corroding the adhesive layer and causing adhesive failure, which tends to lead to vibration failure to affect product performance and use effect. An example of a vaporization device can be seen in document EP3251531.

### SUMMARY

Based on this, it is necessary to provide a microporous vaporization assembly and an electronic vaporization device to solve the problem that a microporous ultrasonic vaporization device is prone to vibration failure.

According to an aspect of the application, a microporous vaporization assembly is provided, the microporous vaporization assembly includes:
a piezoceramic plate which is provided in the piezoceramic plate; and
a vaporization plate which includes a body part and a fence part, the body part being stacked on and bonded to the piezoceramic plate and covering the through hole, a plurality of vaporization holes being provided in an area of the body part corresponding to the through hole.

The fence part is arranged protruding from a surface of the body part stacked with the piezoceramic plate and surrounds an outer circumference of the piezoceramic plate.

In the foregoing microporous vaporization assembly, the vaporization plate is provided with the fence part. The fence part is arranged to protrude from the surface of the body part facing the piezoceramic plate, and to surround the outer circumference of the piezoceramic plate. Thus, the fence part covers a gap between the piezoceramic plate and the body part, so as to prevent a liquid from permeating between the piezoceramic plate and the body part from an edge of the piezoceramic plate during vaporization, and thus, from corroding an adhesive, thereby ensuring the effective bonding between the piezoceramic plate and the vaporization plate and further ensuring that the vaporization plate can effectively vibrate. In addition, the fence part arranged to protrude from the vaporization plate improves the rigidity of the vaporization plate, so that the vaporization plate can be firmly bonded to the piezoceramic plate. In this way, the vaporization plate is prevented from being bent due to its low rigidity after repeated vibrations, and from being separated from the piezoceramic plate due to bending, so that the bonding stability of the vaporization plate and the piezoceramic plate is further improved.

In addition, the fence part is arranged surrounding the outer circumference of the piezoceramic plate, so that an adhesive bonded between the piezoceramic plate and the vaporization plate is prevented from overflowing from an edge of the vaporization plate during compression, heating, and curing, thereby preventing the adhesive from overflowing and affecting a bonding process. In addition, the fence part is arranged on an outer circumference of the vaporization plate, so that the vibration of the vaporization plate can be better restricted in the central area. The central area of the vaporization plate is the area corresponding to the through hole of the piezoceramic plate. The plurality of vaporization holes is provided in the central area of the vaporization plate for vaporizing an aerosol-forming substrate. In this way, the vibration amplitude of an area on the vaporization plate where the vaporization is performed is increased, thereby improving the vaporization effect. Therefore, the microporous vaporization assembly can prevent adhesive failure and adhesive overflow as well as improve the vaporization effect.

In an embodiment, the fence part is integrated with the body part, so that a gap, that allows a liquid to enter a side of the piezoceramic plate, between the fence part and the body part is avoided, thereby further improving a liquid blocking effect of the fence part.

In an embodiment, the fence part is constructed as an annular protrusion sleeved on the outer circumference of the piezoceramic plate at an interval, so as to reserve a particular gap in the outer circumference of the piezoceramic plate to allow the piezoceramic plate to vibrate in a radial direction thereof after being energized, thereby further driving the vaporization holes in the vaporization plate to deform in the axial direction and press and vaporize the aerosol-forming substrate.

In an embodiment, in a radial direction of the annular protrusion, a gap between the annular protrusion and the piezoceramic plate is in a range from 0.01 mm to 1.0 mm.

In an embodiment, a protrusion height of the fence part relative to the body part is less than or equal to a thickness of the piezoceramic plate.

In an embodiment, a ratio of the thickness of the piezoceramic plate to the protrusion height of the fence part relative to the body part is in a range from 1: 1 to 6:1.

In an embodiment, the protrusion height of the fence part relative to the body part is in a range from 0.01 mm to 1.0 mm; and/or the thickness of the piezoceramic plate is in a range from 0.5 mm to 1.0 mm.

In an embodiment, an outer diameter of the piezoceramic plate is in a range from 5 mm to 20 mm; and/or the thickness of the body part is in a range from 0.01 mm to 1 mm.

In an embodiment, the microporous vaporization assembly further includes a bonding layer bonded between the body part and the piezoceramic plate.

According to another aspect of the application, an electronic vaporization device is provided, the electronic vaporization device includes the microporous vaporization assembly according to any of the forgoing embodiments.

### BRIEF DESCRIPTION OF THE DRAWINGS

FIG. 1 is a schematic structural diagram of a microporous vaporization assembly according to an embodiment of the present application;
FIG. 2 is a schematic exploded view of the microporous vaporization assembly shown in FIG. 1;
FIG. 3 is a schematic cross-sectional view of the microporous vaporization assembly shown in FIG. 1;
FIG. 4 is a schematic partial enlarged view of the microporous vaporization assembly shown in FIG. 3;
FIG. 5 is a diagram of vibration modal simulation of an existing microporous vaporization assembly in the related art; and
FIG. 6 is a diagram of vibration modal simulation of the microporous vaporization assembly shown in FIG. 1.

Reference numerals: 100. microporous vaporization assembly; 10. piezoceramic plate; 11. through hole 30. vaporization plate; 31. tab; 32. body part; 33. convex; 34. fence part; and 50. bonding layer.

### DETAILED DESCRIPTION

To make the foregoing objects, features and advantages of the present application more comprehensible, detailed description is made to specific implementations of the present application below with reference to the accompanying drawings. Many details are elaborated in the following description in order to fully understand the present application. However, the present application can be implemented in many other ways different from those described, and similar improvements can be made by technicians in the field without violating the connotation of the present application, so the present application is not limited by specific embodiments disclosed below.

In the description of the present application, it should be understood that, orientation or position relationships indicated by terms such as "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", and "circumferential" are orientation or position relationship shown based on the accompanying drawings, and are merely used for describing the present application and simplifying the description, rather than indicating or implying that the mentioned device or element should have a particular orientation or be constructed and operated in a particular orientation, and therefore, should not be construed as a limitation to the present application.

In addition, the terms "first" and "second" are used for descriptive purposes only and are not to be construed as indicating or implying relative importance or implicitly indicating the number of technical features indicated. Therefore, a feature restricted by "first" or "second" may explicitly indicate or implicitly include at least one of such features. In the description of the present application, unless otherwise explicitly defined, "a plurality of" means at least two, for example, two, three, and the like. In the present application, unless otherwise explicitly specified and defined, terms such as "mounted", "connected", "coupled", and "fixed" should be understood in a broad sense, which may, for example, refer to a fixed connection, a detachable connection, or an integration; or for example, refer to a mechanical connection or an electrical connection; or for example, refer to a direct connection, or an indirect connection through an intermediate medium; or for example, refer to and internal communication between two elements or a mutual action relationship between two elements, unless otherwise explicitly specified. A person of ordinary skill in the art may understand the specific meanings of the foregoing terms in the present application according to specific situations.

In the present application, unless otherwise explicitly specified and defined, a first feature is "on" or "below" a second feature may mean that the first feature and the second feature are in direct, or the first feature and the second feature are in indirect contact through an intermediate medium. In addition, that the first feature is "above", "over", or "on" the second feature may indicate that the first feature is directly above or obliquely above the second feature, or may merely indicate that the horizontal position of the first feature is higher than that of the second feature. That the first feature is "below", "under", and "beneath" the second feature may be that the first feature is directly below or obliquely below the second feature, or may merely indicate that the horizontal position of the first feature is lower than that of the second feature.

It should be noted that, when an element is referred to as "being fixed to" or "being arranged on" another element, the element may be directly on the another element, or an intermediate element may be present. When an element is considered to be "connected to" another element, the element may be directly connected to the another element, or an intermediate element may also be present. The terms "vertical", "horizontal", "upper", "lower", "left", "right", and similar expressions used in this specification are only for purposes of illustration but not indicate a unique implementation.

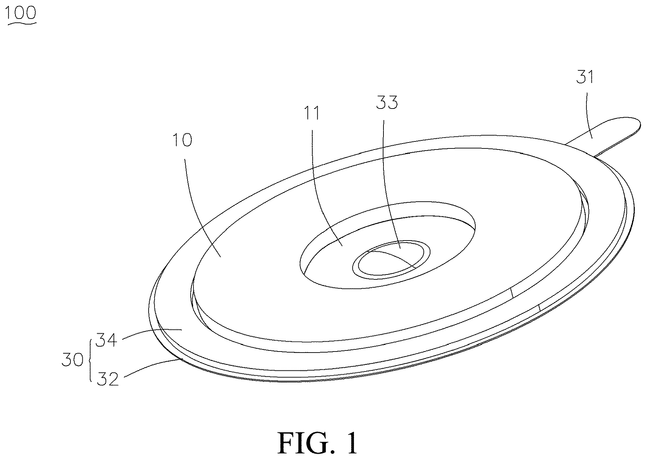

Referring to FIG. 1, an embodiment of the present application provides a microporous vaporization assembly 100 used for vaporizing an aerosol-forming substrate. The microporous vaporization assembly 100 includes a piezoceramic plate 10 and a vaporization plate 30. The vaporization plate 30 and the piezoceramic plate 10 are stacked. A plurality of vaporization holes is provided in the vaporization plate 30. When energized, the the piezoceramic plate 10 vibrates and drives the vaporization plate 30 to vibrate. The vaporization holes continuously and repeatedly deform or vibrate along with the vibration of the vaporization plate 30, so that a liquid aerosol-forming substrate is pressed and broken into fine droplets to form a vaporized aerosol. Generally, the vaporization holes are micron-sized micropores. In some implementations, an aperture of the vaporization hole may be in a range from 1 µm to 20 µm. Preferably, the aperture of the vaporization hole may be in a range from 3 µm to 15 µm.

Further, the piezoceramic plate 10 is provided with a through hole 11. The vaporization plate 30 includes a body part 32. The body part 32 is stacked with and bonded to the piezoceramic plate 10 and covers the through hole 11. A plurality of vaporization holes is provided in an area of the body part 32 corresponding to the through hole 11, so that the vaporization holes are communicated with the through hole 11. When the vaporization plate 30 vibrates, the aerosol-forming substrate is pressed and vaporized by the vaporization holes in the body part 32, so that vaporized droplets are formed. The vaporized droplets may flow out through the through hole 11 in the piezoceramic plate 10.

In some embodiments, the body part 32 and the piezoceramic plate 10 are bonded by a conductive adhesive. The microporous vaporization assembly 100 further includes a positive lead (not shown in the figures) and a negative lead (not shown in the figures). One of the positive lead and the negative lead is fixed and connected to the body part 32, for example, by welding, and the other of the positive lead and the negative lead is fixed and connected to the piezoceramic plate 10, for example, by welding. In this way, the piezoceramic plate 10 of the microporous vaporization assembly 100 is connected to a circuit by the positive lead and the negative lead, so that the piezoceramic plate 10 is energized and vibrates. Optionally, the positive lead or the negative lead is fixed and connected to the body part 32 by welding. Optionally, the body part 32 is provided with a tab 31, which protrudes from the body part 32 in a radial direction, and the positive lead or the negative lead is welded to the tab 31.

Further, generally, a side of the piezoceramic plate 10 away from the vaporization plate 30 is covered with an electrode layer. The electrode layer is a conductive metal layer, for example, the conductive metal layer includes an alloy containing silver. The electrode layer may be connected to the positive lead or the negative lead, and thus, may be further electrically connected to an external power supply, thereby implementing the electrical communication of the piezoceramic plate 10.

Specifically, the area of the vaporization plate 30 provided with the plurality of vaporization holes is configured to protrude in a direction of extending into the through hole 11. That is, a central area of the vaporization plate 30 forms a convex 33 that bends toward the inside of the through hole 11, and the plurality of vaporization holes is provided in the convex 33. In this way, when driven by the piezoceramic plate 10 to vibrate, the vaporization plate 30 may guide the aerosol-forming substrate to flow in a direction of passing through the through hole 11. A protruding direction of the convex 33 is a flowing direction of the substrate.

The vaporization plate 30 further includes the fence part 34. The fence part 34 is arranged to protrude from the surface of the body part 32 on which the piezoceramic plate 10 is stacked, and to surround an outer circumference of the piezoceramic plate 10. Thus, the fence part 34 covers a gap between the piezoceramic plate 10 and the body part 32, so as to prevent a liquid from permeating between the piezoceramic plate 10 and the body part 32 from an edge of the piezoceramic plate 10 during vaporization, and thus, prevent from corroding an adhesive, thereby ensuring the effective bonding between the piezoceramic plate 10 and the vaporization plate 30 and further ensuring that the vaporization plate 30 can effectively vibrate. In addition, the fence part 34 arranged to protrude from the vaporization plate 30 improves the rigidity of the vaporization plate 30, so that the vaporization plate 30 can be firmly bonded to the piezoceramic plate 10. In this way, the vaporization plate 30 is prevented from being bent due to its low rigidity after repeated vibrations, and from being separated from the piezoceramic plate 10 due to bending, so that the bonding stability of the vaporization plate 30 and the piezoceramic plate 10 is further improved.

In addition, the fence part 34 is arranged surrounding the outer circumference of the piezoceramic plate 10, so that an adhesive bonded between the piezoceramic plate 10 and the vaporization plate 30 is prevented from overflowing from an edge of the vaporization plate 30 during compression, heating, and curing, thereby preventing the adhesive from overflowing and affecting a bonding process. In addition, the fence part 34 is arranged on an outer circumference of the vaporization plate 30, so that the vibration of the vaporization plate 30 can be better restricted in the central area. The central area of the vaporization plate 30 is the area corresponding to the through hole 11 of the piezoceramic plate 10. The plurality of vaporization holes is provided in the central area of the vaporization plate 30 for vaporizing the aerosol-forming substrate. In this way, the vibration amplitude of an area on the vaporization plate 30 where the vaporization is performed is increased, thereby improving the vaporization effect. Therefore, the microporous vaporization assembly 100 can prevent adhesive failure and adhesive overflow as well as improve the vaporization effect.

In some embodiments, the body part 32 is integrated with the fence part 34 to avoid a gap between the fence part 34 and the body part 32 that allows a liquid to enter a side of the piezoceramic plate 10, thereby further improving a liquid blocking effect of the fence part 34. Optionally, the vaporization plate 30 is made of any one of stainless steel, a titanium alloy, and a palladium nickel alloy. Optionally, blind holes are opened in raw materials through machining, electroforming or etching, so as to manufacture the body part 32 and the fence part 34 that are integrated.

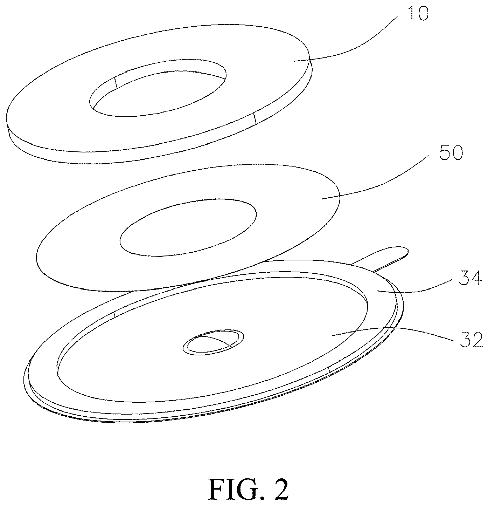

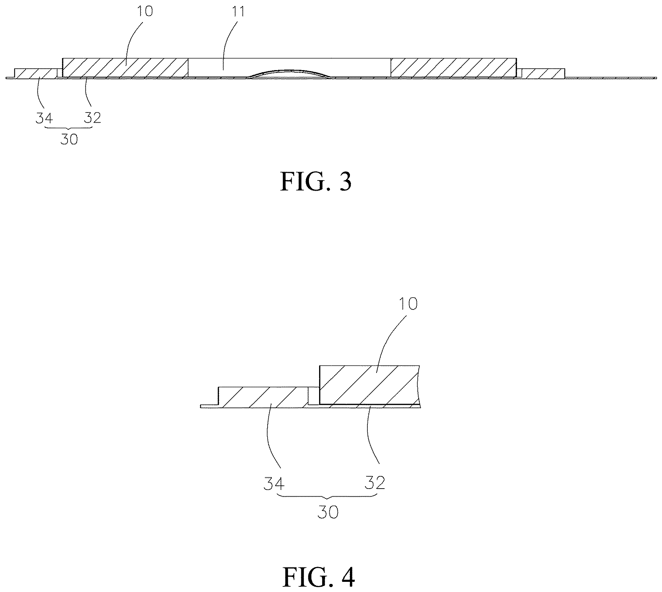

Referring to FIGS. 1 to 3, in some embodiments, the microporous vaporization assembly 100 further includes a bonding layer 50. The bonding layer 50 is bonded between the body part 32 and the piezoceramic plate 10 to bond the vaporization plate 30 to the piezoceramic plate 10. Optionally, the bonding layer 50 is made of epoxy glue or a solid adhesive film. Specifically, in a bonding process, the bonding layer 50 is bonded to a surface of the piezoceramic plate 10 through screen printing (including, but not limited to, steps such as dispensing, coating, and the like); and then the piezoceramic plate 10 with the bonding layer 50 is placed on a portion of the vaporization plate 30 surrounded by the fence part 34, so that the bonding layer 50 on the piezoceramic plate 10 is in contact with the body part 32; finally, the piezoceramic plate 10 and the vaporization plate 30 are laminated and cured at a high temperature by a tools. A curing temperature and a curing duration are determined according to characteristics of the bonding layer 50. In this way, the stacked and bonded piezoceramic plate 10 and the vaporization plate 30 are obtained, thereby obtaining the microporous vaporization assembly 100.

In addition, the fence part 34 is arranged surrounding the outer circumference of the piezoceramic plate 10, so that the adhesive bonded between the piezoceramic plate 10 and the vaporization plate 30 can be prevented from overflowing from an edge of the vaporization plate 30 during compression, heating, and curing, thereby avoiding that the normal use of the tools is affected due to the bonding of the adhesive with the external tools. Therefore, the microporous vaporization assembly 100 according to the embodiment of the present application can prevent adhesive failure and adhesive overflow.

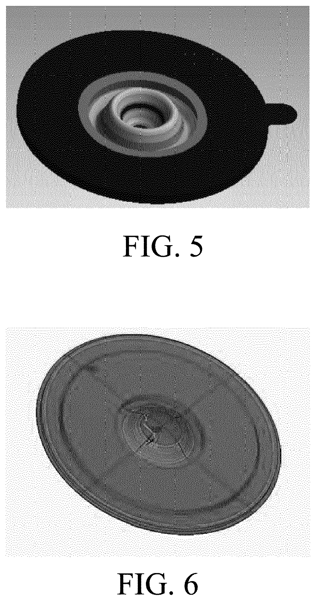

In some embodiments, the fence part 34 is constructed as an annular protrusion which matches the shape of the outer circumference of the piezoceramic plate 10 to block and protect the piezoceramic plate 10 in all directions of the outer circumference of the piezoceramic plate 10, so as to prevent the piezoceramic plate 10 from adhesive failure and adhesive overflow in a bonding process. In addition, the vibration of the vaporization plate 30 is concentrated to the central area through the annular protrusion, thereby improving the vaporization effect. Specifically, referring to FIG. 5 and FIG. 6, FIG. 5is a diagram of vibration modal simulation of an existing microporous vaporization assembly in the related art, and FIG. 6 is a diagram of vibration modal simulation of the microporous vaporization assembly 100. It can be seen from FIG. 6 that the vibration of the microporous vaporization assembly 100 provided in the present application is concentrated in the central area of the vaporization plate 30.

Referring to FIGS. 1, 3, and 4, further, the annular protrusion is sleeved around the outer circumference of the piezoceramic plate 10 at an interval to from a particular gap between the outer circumference of the piezoceramic plate 10 and the annular protrusion, the gap allows the piezoceramic plate 10 to vibrate in a radial direction thereof after being energized, thereby driving the vaporization holes in the vaporization plate 30 to deform in the axial direction and press and vaporize the aerosol-forming substrate.

Optionally, in a radial direction of the annular protrusion, the gap between the annular protrusion and the piezoceramic plate 10 is in a range from 0.01 mm to 1.0 mm, allowing the piezoceramic plate 10 to vibrate in the radial direction thereof.

In some embodiments, a protrusion height of the fence part 34 relative to the body part 32 is less than or equal to the thickness of the piezoceramic plate 10. That is, the fence part 34 is not higher than the piezoceramic plate 10. In this way, the vibration of the vaporization plate 30 is prevented from being hindered by the excessive thickness and weight of the fence part 34, thereby maintaining better vibration and vaporization performance of the vaporization plate 30.

Further, a ratio of the thickness of the piezoceramic plate 10 to the protrusion height of the fence part 34 relative to the body part 32 is in a range from 1: 1 to 6:1. That is, in a case where the fence part 34 has the minimum protrusion height, the thickness of the piezoceramic plate 10 is six times the protrusion height of the fence part 34; and in a case where the fence part 34 has the maximum the protrusion height, the thickness of the piezoceramic plate 10 is equal to the protrusion height of the fence part 34. That is, the protrusion height of the fence part 34 relative to the body part 32 is configured to less than or equal to the thickness of the piezoceramic plate 10.

Optionally, the protrusion height of the fence part 34 relative to the body part 32 is in a range from 0.01 mm to 1.0 mm, the thickness of the piezoceramic plate is in a range from 0.5 mm to 1.0 mm. According to the thickness of the piezoceramic plate 10, the protrusion height of the fence part 34 relative to the body part 32 is appropriately selected so that a liquid is prevent from flowing into a gap on a side of the piezoceramic plate 10 by the fence part 34, thereby preventing the adhesive from corroding. Further, the fence part 34 prevents the adhesive from overflowing from the outer circumference of the vaporization plate 30 during the assembly process, thereby avoiding the influence of the overflow of the adhesive on the assembly process. In addition, the vibration amplitude in the central area of the vaporization plate 30 can be improved by selecting an appropriate thickness for the fence part, so that the vaporization effect of the vaporization plate 30 can be improved. Preferably, the protrusion height of the fence part 34 relative to the body part 32 is in a range from 0.05 mm to 0.8 mm, and the thickness of the piezoceramic plate is in a range from 0.6 to 0.8 mm, so that the microporous vaporization assembly 100 can better prevent adhesive failure and adhesive overflow and have better vibration vaporization performance.

Further, an outer diameter of the piezoceramic plate 10 is in a range from 5 mm to 20 mm, and the thickness of the body part 32 is in a range from 0.01 mm to 1 mm. Specifically, the protrusion height of the fence part 34 relative to the body part 32 is in a range from 0.01 mm to 1.0 mm, and the thickness of the piezoceramic plate is in a range from 0.5 mm to 1.0 mm. In addition, the outer diameter of the piezoceramic plate 10 is in a range from 5 mm to 20 mm, and the thickness of the body part 32 is in a range from 0.01 mm to 1 mm. The size of each component of the microporous vaporization assembly 100 is kept in an appropriate range, thereby ensuring that the microporous vaporization assembly 100 prevents adhesive failure and adhesive overflow and has vibration vaporization performance. The foregoing microporous vaporization assembly 100 includes a piezoceramic plate 10 and a vaporization plate 30. The vaporization plate 30 is stacked on and bonded to the piezoceramic plate. A fence part 34 surrounding an outer circumference of the piezoceramic plate 10 is arranged on the vaporization plate 30 to prevent a liquid from entering a gap between the vaporization plate 30 and the piezoceramic plate 10, and thus, from corroding the adhesive. In addition, the fence part 34 prevents adhesive overflow in a process of assembling the piezoceramic plate 10 and the vaporization plate 30. In addition, the fence part 34 concentrates the vibration of the vaporization plate 30 to the central area provided with vaporization holes, thereby improving the vaporization effect.

Based on the same inventive concept, an embodiment of the present application further provides an electronic vaporization device including the foregoing microporous vaporization assembly 100. The microporous vaporization assembly 100 further includes a piezoceramic plate 10 and a vaporization plate 30. The vaporization plate 30 and the piezoceramic plate 10 are stacked. The vaporization holes are provided in the vaporization plate 30. When energized, the piezoceramic plate 10 vibrates and drives the vaporization plate 30 to vibrate. The vaporization holes continuously and repeatedly deform with the vibration of the vaporization plate 30, so that the liquid aerosol-forming substrate is pressed and broken into fine droplets to form a vaporized aerosol.

Further, the piezoceramic plate 10 is provided with a through hole 11. The vaporization plate 30 includes a body part 32. The body part 32 is stacked on and bonded to the piezoceramic plate 10 and covers the through hole 11. A plurality of vaporization holes is provided in the area of the body part 32 corresponding to the through hole 11, so that the vaporization holes are communicated with the through hole 11. When the vaporization plate 30 vibrates, the aerosol-forming substrate is pressed and vaporized by the vaporization holes in the body part 32, so that the vaporized droplets are med. The vaporized droplets may flow out through the through hole 11 in the piezoceramic plate 10.

The vaporization plate 30 further includes the fence part 34. The fence part 34 is arranged to protrude from the surface of the body part 32 on which the piezoceramic plate 10 is stacked, and to surround an outer circumference of the piezoceramic plate 10. Thus, the fence part 34 covers a gap between the piezoceramic plate 10 and the body part 32, so as to prevent a liquid from permeating between the piezoceramic plate 10 and the body part 32 from an edge of the piezoceramic plate 10 during vaporization, and thus, from corroding the adhesive, thereby ensuring the effective bonding between the piezoceramic plate 10 and the vaporization plate 30 and further ensuring that the vaporization plate 30 can effectively vibrate. In addition, the fence part 34 arranged to protrude from the vaporization plate 30 improves the rigidity of the vaporization plate 30, so that the vaporization plate 30 can be firmly bonded to the piezoceramic plate 10. In this way, the vaporization plate 30 is prevented from being bent due to its low rigidity after repeated vibrations, and from being separated from the piezoceramic plate 10 due to bending, so that the bonding stability of the vaporization plate 30 and the piezoceramic plate 10 is further improved.

In addition, the fence part 34 is arranged surrounding the outer circumference of the piezoceramic plate 10, so that an adhesive bonded between the piezoceramic plate 10 and the vaporization plate 30 is prevented from overflowing from an edge of the vaporization plate 30 during compression, heating, and curing, thereby preventing the adhesive from overflowing and affecting a bonding process. In addition, the fence part 34 is arranged on an outer circumference of the vaporization plate 30, so that the vibration of the vaporization plate 30 can be better restricted in the central area. The central area of the vaporization plate 30 is the area corresponding to the through hole 11 of the piezoceramic plate 10. The plurality of vaporization holes is provided in the central area of the vaporization plate 30 for vaporizing the aerosol-forming substrate. In this way, the vibration amplitude of an area on the vaporization plate 30 where the vaporization is performed is increased, thereby improving the vaporization effect. Therefore, the microporous vaporization assembly 100 can prevent adhesive failure and adhesive overflow as well as improve the vaporization effect.

In some embodiments, the body part 32 is integrated with the fence part 34, to avoid a gap between the fence part 34 and the body part 32 that allows a liquid to enter a side of the piezoceramic plate 10, thereby further improving a liquid blocking effect of the fence part 34. Optionally, the vaporization plate 30 is made of any one of stainless steel, a titanium alloy, and a palladium nickel alloy. Optionally, blind holes are opened in raw materials through machining, electroforming or etching, so as to manufacture the body part 32 and the fence part 34 that are integrated.

The foregoing microporous vaporization assembly 100 includes a piezoceramic plate 10 and a vaporization plate 30. The vaporization plate 30 is stacked on and bonded to the piezoceramic plate. A fence part 34 surrounding an outer circumference of the piezoceramic plate 10 is arranged on the vaporization plate 30 to prevent a liquid from entering a gap between the vaporization plate 30 and the piezoceramic plate 10, and thus, form corroding an adhesive. In addition, the fence part 34 prevents adhesive overflow in a process of assembling the piezoceramic plate 10 and the vaporization plate 30. In addition, the fence part 34 concentrates the vibration of the vaporization plate 30 to the central area provided with vaporization holes, thereby improving the vaporization effect.

The scope of the invention is defined by the claims.

The foregoing embodiments only describe several implementations of the present application. It should be noted that, a person of ordinary skill in the art may further make several variations and improvements without departing from the concept of the present application.

## Claims

1. A microporous vaporization assembly (100), comprising
a piezoceramic plate (10) provided with a through hole (11); **characterized in that** the assembly comprises also
a vaporization plate (30) including a body part (32) and a fence part (34), the body part (32) being stacked on and bonded to the piezoceramic plate (10) and covering the through hole (11), a plurality of vaporization holes being provided in an area of the body part (32) corresponding to the through hole (11),
wherein the fence part (34) is arranged to protrude from a surface of the body part (32) on which the piezoceramic plate (10) is stacked, and to surround an outer circumference of the piezoceramic plate (10).

2. The microporous vaporization assembly (100) according to claim 1, wherein the fence part (34) is integrated with the body part (32).

3. The microporous vaporization assembly (100) according to claim 1, wherein the fence part (34) is constructed as an annular protrusion sleeved on the outer circumference of the piezoceramic plate (10) at an interval.

4. The microporous vaporization assembly (100) according to claim 3, wherein in a radial direction of the annular protrusion, a gap between the annular protrusion and the piezoceramic plate (10) is in a range from 0.01 mm to 1.0 mm.

5. The microporous vaporization assembly (100) according to claim 1, wherein a protrusion height of the fence part (34) relative to the body part (32) is less than or equal to a thickness of the piezoceramic plate (10).

6. The microporous vaporization assembly (100) according to claim 5, wherein a ratio of the thickness of the piezoceramic plate (10) to the protrusion height of the fence part (34) relative to the body part (32) is in a range from 1: 1 to 6:1.

7. The microporous vaporization assembly (100) according to claim 5, wherein the protrusion height of the fence part (34) relative to the body part (32) is in a range from 0.01 mm to 1.0 mm; and/or the thickness of the piezoceramic plate (10) is in a range from 0.5 mm to 1.0 mm.

8. The microporous vaporization assembly (100) according to claim 7, wherein an outer diameter of the piezoceramic plate (10) is in a range from 5 mm to 20 mm; and/or the thickness of the body part (32) is in a range from 0.01 mm to 1 mm.

9. The microporous vaporization assembly (100) according to any one of claims 1 to 8, wherein the microporous vaporization assembly (100) further includes a bonding layer (50) bonded between the body part (32) and the piezoceramic plate (10).

10. An electronic vaporization device, **characterized by** including the microporous vaporization assembly (100) according to any one of claims 1 to 9.

## Patentansprüche

1. Mikroporöse Verdampfungsanordnung (100), aufweisend:
eine piezokeramische Platte (10) mit einem Durchgangsloch (11);
**dadurch gekennzeichnet, dass** die Anordnung außerdem aufweist:
eine Verdampfungsplatte (30) mit einem Körperteil (32) und einem Umrandungsteil (34), wobei das Körperteil (32) stapelförmig auf der piezokeramischen Platte (10) angeordnet und mit dieser verbunden ist und das Durchgangsloch (11) abdeckt, und wobei eine Vielzahl von Verdampfungslöchern in einem dem Durchgangsloch (11) entsprechenden Bereich des Körperteils (32) vorgesehen sind,
wobei das Umrandungsteil (34) derart angeordnet ist, dass es von einer Oberfläche des Körperteils (32), auf dem die piezokeramische Platte (10) stapelförmig angeordnet ist, hervorsteht und einen Außenumfang der piezokeramischen Platte (10) umgibt.

2. Mikroporöse Verdampfungsanordnung (100) nach Anspruch 1, wobei das Umrandungsteil (34) mit dem Körperteil (32) integral ausgebildet ist.

3. Mikroporöse Verdampfungsanordnung (100) nach Anspruch 1, wobei das Umrandungsteil (34) als ein ringförmiger Vorsprung ausgebildet ist, der hülsenartig auf dem Außenumfang der piezokeramischen Platte (10) in einem Abstand angeordnet ist.

4. Mikroporöse Verdampfungsanordnung (100) nach Anspruch 3, wobei in einer radialen Richtung des ringförmigen Vorsprungs ein Spalt zwischen dem ringförmigen Vorsprung und der piezokeramischen Platte (10) in einem Bereich von 0,01 mm bis 1,0 mm liegt.

5. Mikroporöse Verdampfungsanordnung (100) nach Anspruch 1, wobei eine Vorsprunghöhe des Umrandungsteils (34) relativ zum Körperteil (32) kleiner oder gleich einer Dicke der piezokeramischen Platte (10) ist.

6. Mikroporöse Verdampfungsanordnung (100) nach Anspruch 5, wobei das Verhältnis der Dicke der piezokeramischen Platte (10) zur Vorsprunghöhe des Umrandungsteils (34) relativ zum Körperteil (32) in einem Bereich von 1:1 bis 6:1 liegt.

7. Mikroporöse Verdampfungsanordnung (100) nach Anspruch 5, wobei die Vorsprunghöhe des Umrandungsteils (34) relativ zum Körperteil (32) in einem Bereich von 0,01 mm bis 1,0 mm liegt, und/oder die Dicke der piezokeramischen Platte (10) in einem Bereich von 0,5 mm bis 1,0 mm liegt.

8. Mikroporöse Verdampfungsanordnung (100) nach Anspruch 7, wobei ein Außendurchmesser der piezokeramischen Platte (10) in einem Bereich von 5 mm bis 20 mm liegt; und/oder die Dicke des Körperteils (32) in einem Bereich von 0,01 mm bis 1 mm liegt.

9. Mikroporöse Verdampfungsanordnung (100) nach einem der Ansprüche 1 bis 8, wobei die mikroporöse Verdampfungsanordnung (100) ferner eine Verbindungsschicht (50) aufweist, die zwischen dem Körperteil (32) und der piezokeramischen Platte (10) verbunden ist.

10. Elektronische Verdampfungsvorrichtung, **dadurch gekennzeichnet, dass** sie die mikroporöse Verdampfungsanordnung (100) nach einem der Ansprüche 1 bis 9 aufweist.

## Revendications

1. Ensemble vaporisation microporeuse (100), comprenant
une plaque de céramique piézo (10) pourvue d'un orifice traversant (11) ; **caractérisé en ce que** l'ensemble comprend également
une plaque de vaporisation (30) incluant une pièce de corps (32) et une pièce de clôture (34), la pièce de corps (32) étant empilée sur et liée à la plaque de céramique piézo (10) et recouvrant l'orifice traversant (11), une pluralité d'orifices de vaporisation étant pourvue dans une zone de la pièce de corps (32) correspondant à l'orifice traversant (11),
dans lequel la pièce de clôture (34) est agencée pour faire saillie depuis une surface de la pièce de corps (32) sur laquelle la plaque de céramique piézo (10) est empilée, et pour entourer une circonférence externe de la plaque de céramique piézo (10).

2. Ensemble vaporisation microporeuse (100) selon la revendication 1, dans lequel la pièce de clôture (34) est intégrée à la pièce de corps (32).

3. Ensemble vaporisation microporeuse (100) selon la revendication 1, dans lequel la pièce de clôture (34) est construite en tant que protubérance annulaire emmanchée sur la circonférence externe de la plaque de céramique piézo (10) avec un intervalle.

4. Ensemble vaporisation microporeuse (100) selon la revendication 3, dans lequel dans une direction radiale de la protubérance annulaire, un écart entre la protubérance annulaire et la plaque de céramique piézo (10) dans une plage de 0,01 mm à 1,0 mm.

5. Ensemble vaporisation microporeuse (100) selon la revendication 1, dans lequel une hauteur de protubérance de la pièce de clôture (34) par rapport à la pièce de corps (32) est inférieure ou égale à une épaisseur de la plaque de céramique piézo (10).

6. Ensemble vaporisation microporeuse (100) selon la revendication 5, dans lequel un rapport de l'épaisseur de la plaque de céramique piézo (10) à la hauteur de protubérance de la pièce de clôture (34) par rapport à la pièce de corps (32) se situe dans une plage de 1:1 à 6:1.

7. Ensemble vaporisation microporeuse (100) selon la revendication 5, dans lequel la hauteur de protubérance de la pièce de clôture (34) par rapport à la pièce de corps (32) se situe dans une plage de 0,01 mm à 1,0 mm ; et/ou l'épaisseur de la plaque de céramique piézo (10) se situe dans une plage de 0,5 mm à 1,0 mm.

8. Ensemble vaporisation microporeuse (100) selon la revendication 7, dans lequel un diamètre externe de la plaque de céramique piézo (10) se situe dans une plage de 5 mm à 20 mm ; et/ou l'épaisseur de la pièce de corps (32) se situe dans une plage de 0,01 mm à 1 mm.

9. Ensemble vaporisation microporeuse (100) selon l'une quelconque des revendications 1 à 8, dans lequel l'ensemble vaporisation microporeuse (100) inclut en outre une couche de liaison (50) liée entre la pièce de corps (32) et la plaque de céramique piézo (10).

10. Dispositif de vaporisation électronique, **caractérisé en ce qu'**il inclut l'ensemble vaporisation microporeuse (100) selon l'une quelconque des revendications 1 à 9.
